(19) Europäisches Patentamt
European Patent Office
Office européen des brevets

(11) **EP 3 213 757 A1**

(12) **EUROPEAN PATENT APPLICATION**
published in accordance with Art. 153(4) EPC

(43) Date of publication:
**06.09.2017 Bulletin 2017/36**

(51) Int Cl.:
***A61K 33/06*** *(2006.01)*     ***A61K 33/14*** *(2006.01)*
***A61P 9/12*** *(2006.01)*

(21) Application number: **15854802.4**

(22) Date of filing: **29.10.2015**

(86) International application number:
**PCT/KR2015/011492**

(87) International publication number:
**WO 2016/068615 (06.05.2016 Gazette 2016/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **29.10.2014 KR 20140148415**

(71) Applicant: **Aribio Inc.**
**Seoul 07286 (KR)**

(72) Inventors:
• **CHOUNG, Jai Jun**
**Yongin-si**
**Gyeonggi-do 16930 (KR)**
• **SUNG, Soo Hyun**
**Seoul 05242 (KR)**
• **KIM, Jong Kyu**
**Yongin-si**
**Gyeonggi-do 17079 (KR)**
• **SHIN, Chang Ho**
**Ansan-si**
**Gyeonggi-do 15337 (KR)**

(74) Representative: **Kilger, Ute**
**Boehmert & Boehmert**
**Anwaltspartnerschaft mbB**
**Pettenkoferstrasse 22**
**80336 München (DE)**

(54) **COMPOSITION, CONTAINING HIGH-HARDNESS MINERAL WATER PREPARED FROM SALTY UNDERGROUND WATER OR DEEP-SEA WATER, FOR PREVENTING OR ALLEVIATING DECREASE IN BLOOD PRESSURE OR SYMPTOMS RELATED THERETO**

(57) The present invention relates to a composition, containing, as active ingredient, high-hardness mineral water prepared from salty underground water or deep-sea water, for preventing or alleviating a decrease in blood pressure or symptoms related thereto. According to the present invention, the high-hardness mineral water prepared from salty underground water or deep-sea water, especially, prevents a rapid decrease in blood pressure or mitigates the degree of decrease in blood pressure, during or after excercise, and thus can be used to prevent or alleviate symptoms related to a rapid decrease in blood pressure during excercise.

## Description

## Technical Field

The present invention relates to a composition containing high-hardness mineral water, prepared from salty underground water or deep sea water, as an active ingredient for preventing or alleviating a decrease in blood pressure or symptoms associated with a decrease in blood pressure.

## Background Art

Low blood pressure means that the blood pressure is lower than normal (systolic blood pressure = 120 mmHg, diastolic blood pressure = 80 mmHg) when the pressure of the forearm artery is generally measured by a blood pressure meter. Low blood pressure during or after exercise is defined by the systolic blood pressure during exercise being decreased by 20 mmHg or more compared with that before exercise. When the blood pressure is decreased, various prognoses and symptoms may occur depending on the degree of blood pressure decrease. However, the degree of blood pressure decrease characterized as low blood pressure may vary from individual to individual.

Symptoms associated with decreased blood pressure include fainting, dizziness, headache, nausea, numbness, systemic weakness, insomnia, brachycardia (slow pulse), constipation, pale skin, temporary visual and hearing impairment, shock, arrhythmia, and the like. A sudden decrease in blood pressure during or after exercise is accompanied by severe nervous breakdown, dizziness accompanied by fainting, and wobbling, as well as a phenomenon in which objects appear shaky. However, during or after exercise, a decrease of 8 SBP/9 DBP mmHg for a healthy person without a specific disease, a decrease of 14 SBP/9 DBP mmHg for a person with borderline hypertension, and a decrease of 10 SBP/7 DBP for a person with hypertension are evaluated as a positive effect of exercise.

In general, the blood pressure increases temporarily ($\leq$ 5 minutes) in the early stage of exercise (including all the resistance exercise and endurance exercise) and decreases continuously from 10 minutes after exercise to the end of exercise, and an increase in dehydration together with an increase in exercise duration may cause a sudden decrease in blood pressure. During exercise, a rapid decrease in blood pressure may cause aortic valvular disorder or serious heart disease. Therefore, during exercise, a decrease in blood pressure needs to be closely examined, and various efforts need to be made to prevent a reduction in oxygen supply and the syncope in case of hypotension. In addition, a decrease in blood pressure needs to occur within an appropriate range during exercise, and a decrease in blood pressure after cessation of exercise may be maintained for a long time (10-12 hours).

Throughout the entire specification, many papers and patent documents are referenced and their citations are represented. The disclosures of the cited papers and patent documents are entirely incorporated by reference into the present specification, and the level of the technical field within which the present invention falls and the details of the present invention are thus explained more clearly.

## Detailed Description of the Invention

## Technical Problem

The present inventors have made a research effort to develop a composition capable of preventing and alleviating a decrease in blood pressure, particularly, a sudden decrease in blood pressure during or after exercise. As a result, the present inventors have verified that in case of a marathoner consuming, as drinking water, high-hardness mineral water, prepared from salty underground water or deep sea water, at the time of 100-km ultramarathon, his/her blood pressure was maintained at the normal blood pressure level (120/80 mmHg), and the decrease in blood pressure was also remarkably smaller than that in a marathoner consuming general purified water, and thus have confirmed that the high-hardness mineral water can be used to prevent and alleviate a decrease in blood pressure, and blood pressure decrease-associated symptoms, and therefore have completed the present invention.

Accordingly, one aspect of the present invention is to provide a composition for preventing or alleviating a decrease in blood pressure.

Another aspect of the present invention is to provide a method for preventing or alleviating a decrease in blood pressure.

Still another aspect of the present invention is to provide a composition for preventing or alleviating blood pressure decrease-associated symptoms.

Still another aspect of the present invention is to provide a method for preventing or alleviating blood pressure decrease-associated symptoms.

Other purposes and advantages of the present invention will become more obvious with the following detailed description of the invention, claims, and drawings.

**Technical Solution**

In accordance with an aspect of the present invention, there is provided a pharmaceutical composition a composition for preventing or alleviating a decrease in blood pressure, the composition containing, as an active ingredient, mineral water having a hardness value of 100-2000 prepared from salty underground water or deep sea water.

In accordance with another aspect of the present invention, there is provided a method for preventing or alleviating a decrease in blood pressure, the method including a step of administering a composition to a subject in need thereof, the composition containing, as an active ingredient, mineral water having a hardness value of 100-2000 prepared from salty underground water or deep sea water.

The present inventors have made a research effort to develop a composition capable of preventing and alleviating a decrease in blood pressure, particularly, a sudden decrease in blood pressure during or after exercise. As a result, the present inventors have verified that in case of a marathoner consuming, as drinking water, high-hardness mineral water, prepared from salty underground water or deep sea water, at the time of 100-km ultramarathon, his/her blood pressure was maintained at the normal blood pressure level (120/80 mmHg), and the decrease in blood pressure was also remarkably smaller than that in a marathoner consuming general purified water, and thus have confirmed that the high-hardness mineral water can be used to prevent and alleviate a decrease in blood pressure, and blood pressure decrease-associated symptoms.

As used herein, the term "a decrease in blood pressure" refers to a state in which the blood pressure is lowered compared with the normal blood pressure (there are individual differences, but about 120/80 mmHg), or a state in which the blood pressure is lowered to a blood pressure level at which symptoms occur, such as dizziness, headache, fainting, nervous breakdown, wobbling, nausea, numbness, systemic weakness, temporary visual and hearing impairment, constipation, insomnia, slow pulse, pale skin, shock, and arrhythmia. According to the definition, the term "prevention" refers to preventing (suppressing) the blood pressure from dropping below a normal blood pressure by the intake of the composition of the present invention, reducing the width of blood pressure decrease (preventing a rapid decrease in blood pressure) by the intake of the composition of the present invention, and preventing the blood pressure from dropping to a blood pressure level accompanied by the above symptoms by the intake of the composition of the present invention; and the term "alleviation" refers to increasing the blood pressure to a normal blood pressure level, returning the blood pressure to a level at which symptoms accompanying a decrease in blood pressure do not occur, or reducing the number or occurrence or intensity of symptoms accompanying such a decrease in blood pressure.

According to one embodiment of the present invention, a decrease in blood pressure is a decrease in blood pressure during or after exercise. For example, the exercise may be aerobic exercise which lasts for 10 minutes or more. Such aerobic exercise includes ball games such as soccer and basketball, walking, running (for example, long distance running such as a marathon or ultramarathon), cycling, mountaineering, swimming, running on a treadmill, and the like.

As used herein, the term "a decrease in blood pressure during or after exercise" refers to a state in which the systolic blood pressure during or after exercise is lowered by 14 mmHg or more compared with that before exercise, or a state in which the blood pressure is lowered to a blood pressure level at which symptoms, such as dizziness, headache, fainting, nervous breakdown, wobbling, nausea, numbness, systemic weakness, temporary visual and hearing impairment, constipation, insomnia, slow pulse, pale skin, shock, and arrhythmia, occur by a decreased blood pressure due to exercise.

According to one embodiment of the present invention, the composition of the present invention prevents a rapid decrease in blood pressure (systolic or diastolic blood pressure). That is, the composition of the present invention reduces a width of blood pressure decrease. For example, such a rapid decrease in blood pressure is a decrease in blood pressure during or after exercise.

As used herein, the term "salty underground water" refers to underground water in bedrock aquifers, containing more than a predetermined amount of dissolved solids, such as salt, and means raw water which is to be drunk in a natural state in which the stability of the water quality can be continuously maintained; and the term "deep ocean water" refers to water of the sea that is at a depth of 200 m or more where sunlight does not reach.

The high-hardness mineral water contained as an active ingredient in the present invention can be prepared using salty underground water, and any salty underground water may be used in the preparation of the high-hardness mineral water without limitation. For example, high-hardness mineral water may be prepared by using underground water in bedrock aquifers, in which the total content of dissolved solids, such as salt dissolved in water, is 2000 mg/$\ell$ or more.

As used herein, the term "hardness" refers to water strength which is generated by divalent metal cations, such as $Ca^{2+}$ and $Mg^{2+}$, dissolved in water, and expressed as a value in terms of $CaCO_3$.

According to an embodiment of the present invention, the hardness may be calculated by the following equation

[Equation 1]

$$\text{Water hardness (CaCO}_3 \text{ mg/}\ell) = 2.5 \times [\text{Ca}^{2+} \text{ concentration (mg/}\ell)] + 4.1 \times [\text{Mg}^{2+} \text{ concentration (mg/}\ell)]$$

The mineral water of the present invention has a hardness (mg/$\ell$ as CaCO$_3$) of 100-2000. The mineral water with a hardness of 100-2000 may contain 15-500 mg/$\ell$ magnesium, 5-170 mg/$\ell$ calcium, and 4.5-150 mg/$\ell$ potassium.

The mineral water of the present invention may have various hardness values within the above hardness range. According to an embodiment of the present invention, the hardness of the mineral water is 100-1900, 100-1800, 100-1700, 100-1600, 100-1500, 100-1400, 100-1300, 100-1200, 100-1100 or 100-1000; according to another embodiment, the hardness of the mineral water is 200-1900, 200-1800, 200-1700, 200-1600, 200-1500, 200-1400, 200-1300, 200-1200, 200-1100 or 200-1000; according to still another embodiment, the hardness of the mineral water is 300-1900, 300-1800, 300-1700, 300-1600, 300-1500, 300-1400, 300-1300, 300-1200, 300-1100 or 300-1000; according to still another embodiment, the hardness of the mineral water is 400-1900, 400-1800, 400-1700, 400-1600, 400-1500, 400-1400, 400-1300, 400-1200, 400-1100 or 400-1000; according to still another embodiment, the hardness of the mineral water is 500-1900, 500-1800, 500-1700, 500-1600, 500-1500, 500-1400, 500-1300, 500-1200, 500-1100 or 500-1000; and according to still another embodiment, the hardness of the mineral water is 600-1900, 600-1800, 600-1700, 600-1600, 600-1500, 600-1400, 600-1300, 600-1200, 600-1100 or 600-1000.

Of the mineral water having the above hardness values, representatively, mineral water with a hardness of 100 contains 15-25 mg/$\ell$ magnesium, 5-8.5 mg/$\ell$ calcium, and 4.5-7.5 mg/$\ell$ potassium; mineral water with a hardness of 300 contains 45-75 mg/$\ell$ magnesium, 15-25.5 mg/$\ell$ calcium, and 13.5-22.5 mg/$\ell$ potassium; mineral water with a hardness of 700 contains 105-175 mg/$\ell$ magnesium, 35-59.5 mg/$\ell$ calcium, and 31.5-52.5 mg/$\ell$ potassium; and mineral water with a hardness of 1000 contains 150-250 mg/$\ell$ magnesium, 50-85 mg/$\ell$ calcium, and 45-75 mg/$\ell$ potassium (containing 15-25 mg/$\ell$ magnesium, 5-8.5 mg/$\ell$ calcium, and 4.5-7.5 mg/$\ell$ potassium per hardness of 100).

According to an embodiment of the present invention, the composition of the present invention contains mineral water with a hardness of 100-1200 containing 15-300 mg/$\ell$ magnesium, 5-102 mg/$\ell$ calcium, and 4.5-90 mg/$\ell$ potassium.

According to an embodiment of the present invention, the composition of the present invention contains mineral water with a hardness of 200-1200 containing 30-300 mg/$\ell$ magnesium, 10-102 mg/$\ell$ calcium, and 9-90 mg/$\ell$ potassium.

According to an embodiment of the present invention, the composition of the present invention contains mineral water with a hardness of 300-1200 containing 45-300 mg/$\ell$ magnesium, 15-102 mg/$\ell$ calcium, and 13.5-90 mg/$\ell$ potassium.

According to another embodiment of the present invention, the composition of the present invention contains mineral water with a hardness of 400-1200 containing 60-300 mg/$\ell$ magnesium, 20-102 mg/$\ell$ calcium, and 18-90 mg/$\ell$ potassium.

According to still another embodiment of the present invention, the composition of the present invention contains mineral water with a hardness of 500-1200 containing 75-300 mg/$\ell$ magnesium, 25-102 mg/$\ell$ calcium, and 22.5-90 mg/$\ell$ potassium.

According to still another embodiment of the present invention, the composition of the present invention contains mineral water with a hardness of 600-1200 containing 90-300 mg/$\ell$ magnesium, 30-102 mg/$\ell$ calcium, and 27-90 mg/$\ell$ potassium.

Meanwhile, the composition of the present invention may be prepared in various forms. For example, the composition of the present invention may be prepared in a form of drinking water, tea, a sports drink, an isotonic drink or an energy drink. For example, the composition of the present invention is prepared as drinking water and thus can be conveniently taken for the prevention and alleviation of low blood pressure or low blood pressure-associated symptoms. On the other hand, various drinks may be prepared by further adding citric acid, liquid fructose, sugar, glucose, additional minerals (for example, phosphate, iron, and copper), various vitamins, acetic acid, malic acid, juice, and/or various plant extracts (for example, plant extracts containing polyphenols), in addition to high-hardness mineral water as an active ingredient.

The mineral water of the present invention has a hard hardness, and is characterized by containing large quantities of magnesium, calcium, and potassium ions. However, the preparation of the mineral water containing such ions in large quantities has the following problems. As for a first problem, in cases where a mineral concentrate is added in large quantities to desalted water in order to increase the mineral content in drinking water, the drinking water contains large quantities of sulfate and chlorine ions, which degrade the texture, as well as cations, such as magnesium or potassium ions. As for a second problem, in cases where calcium salts separated from concentrated water are added to desalted water in order to supply calcium, the texture of drinking water deteriorates due to the addition of salt components and calcium carbonate contained in the calcium salts, and the substantial supply efficiency of calcium is low due to the low solubility of calcium salts. Therefore, the preparation of the high-hardness mineral water is not easy due to the above problems.

Therefore, the composition of the present invention may contain mineral water having a hardness of 100-2000, the mineral water being prepared by mixing desalted water, which is obtained by performing a desalting treatment on salty underground water or deep sea water, with (i) a mineral concentrate obtained by separating calcium salt crystals and salt from concentrated water obtained by performing a desalting treatment on deep sea water or salty underground water; and (ii) calcium salts obtained by removing, from the calcium salt crystals separated from the concentrated water, salt and calcium carbonate attached to the calcium salt crystals.

Respective steps will be described below.

The desalting treatment for separating desalted water and concentrated water from salty underground water or deep sea water may be performed by employing any technique known in the art. Exemplary techniques for the desalting treatment may be an evaporation method, a seawater freezing method, a reverse osmosis method, an ion exchange resin method, an electrodialysis method, and the like. According to an embodiment of the present invention, through the desalting treatment, raw water is allowed to pass through a reverse osmotic membrane to be separated into concentrated water containing ion components and desalted water from which ion components are removed. The raw water may be used after having gone through a pretreatment process, such as filtration, for removing floating materials. The pretreatment process is conducted to remove impurities that may cause a membrane blockage in the reverse osmosis filtration, and typically, microfiltration or ultrafiltration may be conducted.

In the present invention, the desalted water may be obtained from the raw water by a desalting treatment with two or more steps. For example, the raw water is passed through a first reverse osmosis membrane to obtain first concentrated water and first desalted water, and the first desalted water is passed through a second reverse osmosis membrane to obtain second concentrated water and second desalted water. After that, calcium salts and a mineral concentrate, which are separated from the first concentrated water, are added to the second desalted water to prepare high-hardness mineral water.

The concentrated water contains calcium salts, such as calcium carbonate, calcium sulfate, and calcium chloride, sodium chloride, and minerals in large quantities. In the present invention, the calcium salts and salt are gradationally separated from the concentrated water separated from the raw water.

In the present invention, as for the separation of calcium salts and salt, in order to precipitate calcium salt crystals and salt from the concentrated water, the concentrated water is heated and concentrated such that the concentrated water has an appropriate specific gravity value (a ratio of concentrated water density to water density under the same temperature and pressure), thereby separating the precipitated calcium salt crystals and salt.

According to one embodiment of the present invention, as for the separation of calcium salts, the concentrated water is heated and concentrated such that the specific gravity (a ratio of concentrated water density to water density under the same temperature and pressure) is 1.11 or more, thereby separating precipitated calcium salt crystals. The preferable specific gravity of the concentrated water for the separation of calcium salts is, but is not limited to, 1.11-1.23. The separation of calcium salts may be conducted using a mesh net, and the separation may be conducted using preferably a 300- to 350-mesh net, and more preferably a 300-mesh net.

According to another embodiment of the present invention, the concentrated water is heated and concentrated such it has a specific gravity of 1.18, thereby first separating the precipitated calcium salts, and the filtrate is heated and concentrated such that it has a specific gravity of 1.19-1.23, thereby removing residual calcium salts.

According to still another preferable embodiment of the present invention, the concentrated water is heated such that it has a specific gravity of 1.23, and then is allowed to stand, thereby extracting calcium salts deposited on the bottom.

According to an embodiment of the present invention, as for the separation of salt, the concentrated water is heated and concentrated such that it has a specific gravity of 1.24 or more, thereby separating the precipitated salt. The preferable specific gravity of the concentrated water for the separation of the salt is, but is not limited to, 1.24-1.32. Since a mineral concentrate (liquid) and salt (solid) are present together in the heated concentrated water, the salt may be separated at the time of centrifugation or by using a dehydrator. The separated salt may be processed into mineral salt through an additional purification process.

In the present invention, the heating of the concentrated water may be slowly conducted simultaneously with stirring.

In the present invention, the concentrated water, from which the calcium salts and the salt have been removed, may be further heated and concentrated in order to concentrate mineral components.

According to an embodiment of the present invention, negative ions and impurities are removed from the mineral concentrate, from which calcium salts and salt components have been removed and which is to be mixed with desalted water, by a method for improving quality of a mineral concentrate, the method including: (a) filtering the mineral concentrate through a filter having a physical adsorbent; (b) re-filtering the filtered mineral concentrate through a filter having a physical adsorbent; and (c) filtering the mineral concentrate, which has been filtered in step (b), through a hollow fiber membrane filter having a plurality of pores. The removal of the impurities is absolutely necessary for the preparation of drinking water, and the removal of negative ions is required to improve a feeling of refreshment of mineral water. The present procedure is characterized by filtering the mineral concentrate through a filter having a physical adsorbent and then re-filtering the filtered mineral concentrate through a filter having a physical adsorbent. For the filters used for the filtering and re-filtering, the same filter may be reused, or separate filters may be used. In addition, the filter for the re-filtering may be different from the filter used for first filtering with respect to the filter length, constituent elements, kind of adsorbent, and/or size of micropores of the adsorbent.

In the present invention, when the mineral concentrate passes through the filter having a physical adsorbent, negative ions (especially, chlorine ions and sulfate ions) and impurities (especially, silt) in the mineral concentrate are removed by being adsorbed in the plurality of micropores of the adsorbent, and the removing efficiency of negative ions and impurities is maximized through re-filtering using the filter having a physical adsorbent (first filtering). After that, the first-filtered mineral concentrated water is second filtered through a hollow fiber membrane filter, and thus, the removing efficiency of negative ions and impurities is further improved (second filtering).

In the present invention, any filter that has a physical adsorbent capable of adsorbing impurities and negative ions present in the solution may be used without limitation. According to an embodiment of the present invention, the physical adsorbent is activated carbon, diatomite, zeolite, silica gel, starch, bentonite or alumina, and is more preferably activated carbon.

In the present invention, an appropriate activated carbon filter may be selectively used depending on the amount of the mineral concentrate. For example, for the treatment of 100 ℓ of the mineral concentrate, an 8- to 12-inch activated carbon filter is preferable, and for the treatment of 200 ℓ of the mineral concentrate, a 18-to 22-inch activated carbon filter is preferable. More preferably, a 10-inch activated carbon filter is used for treating 100 ℓ of the mineral concentrate, and a 20-inch activated carbon filter is used for treating 200 ℓ of the mineral concentrate.

In the present invention, the concentrated water, which has been first filtered through the filter having a physical adsorbent, is second filtered through a hollow fiber membrane filter, thereby further filtering out negative ions, and impurities containing microorganisms and silt, and allowing the mineral components in the mineral concentrate to pass through the hollow fiber membrane filter. As the hollow fiber membrane filter, any micro-filter that has a plurality of pores may be used without limitation, and the pores have a diameter of 0.01-0.5 μm, preferably 0.05-0.5 μm, and more preferably 0.1-0.5 μm.

Preferably, the hollow fiber membrane filter is a sterile filter.

According to an embodiment of the present invention, the silt and negative ions in the mineral concentrate are removed by the filtering, and preferably, silt, chlorine ions, and sulfate ions are removed.

In the present invention, in order to improve the removing efficiency of impurities and negative ions, the circulation procedure may be repeatedly performed: filtering the mineral concentrate through a filter having a physical adsorbent → re-filtering the filtered concentrate through a filter having a physical adsorbent → re-re-filtering the re-filtered concentrate. The number of repetitions is preferably once or more, more preferably 1-5 times, and still more preferably 1-4 times.

In the present invention, the mineral concentrate may be supplied into the filter by a supply unit at an appropriate rate or an appropriate flow rate. The supply unit preferably employs a pump, and more preferably a controlled volume pump that can deliver the mineral concentrate to the filter at a constant rate (or flow rate).

According to an embodiment of the present invention, the removal of salt and calcium carbonate from the calcium salt crystals separated from the concentrated water may be conducted by (i) feeding the calcium salt crystals, which have been separated from the concentrated water, into a container, which contains hot water and is equipped with a 300- to 350-mesh net; and (ii) separating the calcium salt crystals which do not pass through the net. Here, the salt attached onto the calcium salt crystals is dissolved in the hot water, and the calcium carbonate passes through the mesh net and then is deposited on the bottom in the container. Since the calcium salt crystals separated from the concentrated water have been separated from the concentrated water containing large quantities of salt components, the calcium salt crystals necessarily contain the concentrated water. Therefore, in cases where the calcium salt crystals, without the removal of the salt components, are added to the desalted water, the salt components of the concentrated water degrade the texture of the mineral water. Moreover, the texture of the mineral water is degraded due to calcium carbonate. Thus, in the present invention, a purification procedure is performed to remove the calcium carbonate and salt (concentrated water) attached to the calcium salt crystals.

According to an embodiment of the present invention, in step (i), the container is slowly shaken after the calcium salt crystals are fed into the container.

According to an embodiment of the present invention, for the mesh net in step (i), a net is used that has an equal or higher standard than the mesh net which has been used when the calcium salt crystals are extracted. More preferably, a 300-mesh (300 holes per inch) to 350-mesh net is used, and still more preferably, a 300-mesh net is used.

The temperature of the solution in the container is set to a temperature at which the calcium salts can be precipitated as crystals (the calcium salts can exist as crystals). When the temperature of the solution is lower than the above temperature, the calcium salts are dissolved in water, thereby lowering the filtering efficiency of calcium carbonate by the mesh net. The temperature of the solution may be set to 60-100 °C, preferably 65-100 °C, and more preferably 70-100 °C. The reason is that, since the calcium salts are extracted at 70-100 °C, the same conditions are set.

The calcium salt crystals separated in step (ii) may be dried by natural drying, a dry oven, a microwave oven, or the like, before being stored. Preferably, the calcium salt crystals are dried using a dry oven or a microwave oven. After that, the stored calcium salts may be added to desalted water to supply calcium.

Through the above method, high-hardness mineral water that contains large quantities of magnesium, calcium, and potassium and has an excellent texture can be prepared, and the prepared high-hardness mineral water can be used as an active ingredient of the composition of the present invention for the prevention and alleviation of low blood pressure and low blood pressure-associated symptoms.

As used herein, the term "subject" includes, but is not limited to, a human being, mouse, rat, guinea pig, dog, cat, horse, cow, pig, monkey, chimpanzee, beaver or rhesus monkey. Specifically, the subject of the present invention is a human being.

In accordance with still another aspect of the present invention, there is provided a composition containing the above-described composition as an active ingredient for preventing or alleviating blood pressure decrease-associated symptoms caused by a decrease in blood pressure, the symptoms being selected from the group consisting of dizziness, headache, fainting, wobbling, nausea, numbness, systemic weakness, temporary visual and hearing impairment, nervous breakdown, constipation, insomnia, slow pulse, pale skin, shock, and arrhythmia.

In accordance with still another aspect of the present invention, there is provided a method for preventing or alleviating blood pressure decrease-associated symptoms caused by a decrease in blood pressure, the method including a step of administering a composition to a subject in need thereof, the composition containing, as an active ingredient, mineral water having a hardness value of 100-2000 prepared from salty underground water or deep sea water, the symptoms being selected from the group consisting of dizziness, headache, fainting, wobbling, nausea, numbness, systemic weakness, temporary visual and hearing impairment, nervous breakdown, constipation, insomnia, slow pulse, pale skin, shock, and arrhythmia.

Since the composition for preventing or alleviating blood pressure decrease-associated symptoms contains, as an active ingredient, the foregoing composition for preventing or alleviating a decrease in blood pressure, descriptions of overlapping contents between the two are omitted to avoid excessive complication of the specification due to repetitive descriptions thereof.

Here, dizziness, headache, fainting, wobbling, nausea, numbness, systemic weakness, temporary visual or hearing impairment (including a phenomenon in which objects appear shaky), nervous breakdown, constipation, insomnia, slow pulse, pale skin, shock, and arrhythmia are physical symptoms caused by decreased blood pressure, and the high-hardness mineral water prepared from the salty underground water or the deep sea water has the effect of preventing the dropping of the blood pressure or reducing the degree of blood pressure dropping, thereby preventing or alleviating the above blood pressure decrease-associated symptoms. The intake of the composition of the present invention can prevent the occurrence of the above symptoms due to a decrease in blood pressure during or after exercise or reduce the number or occurrence or intensity of the symptoms for one example, and can prevent the occurrence of the above-described symptoms caused by orthostatic hypotension or reduce the number or occurrence or intensity of the symptoms for another example.

**Advantageous Effects**

Features and advantages of the present invention are summarized as follows:

(i) The present invention relates to a composition containing high-hardness mineral water prepared from salty underground water or deep sea water as an active ingredient for preventing or alleviating a decrease in blood pressure or symptoms associated with a decrease in blood pressure.

(ii) According to the present invention, the high-hardness mineral water prepared from salty underground water or deep sea water exhibits an effect of preventing a sudden dropping of the blood pressure, especially, during or after exercise or reducing the degree of blood pressure dropping, and thus, the high-hardness mineral water can be used for the prevention and alleviation of symptoms associated with a sudden decrease in blood pressure during and after exercise.

**Brief Description of the Drawings**

FIG. 1 shows a change in systolic blood pressure in an active group and a control group.
FIG. 2 shows a change in diastolic blood pressure in an active group and a control group.

## Mode for Carrying Out the Invention

Hereinafter, the present invention will be described in detail with reference to examples. These examples are only for illustrating the present invention more specifically, and it will be apparent to those skilled in the art that the scope of the present invention is not limited by these examples.

## EXAMPLE

**Verification of effect of preventing blood pressure decrease by intake of high-hardness mineral water**

## Methods

It was examined whether a sudden decrease in blood pressure during or after exercise was prevented by the intake of high-hardness mineral water at the time of a 100 km ultramarathon. Specific experimental methods were as follows. Of the participants participating in the 100 km ultramarathon, 23 subjects in a control group and 30 subjects in an experimental group were selected in advance. The experimental group and the control group were distinguished from each other by wearing LED bands (Belt ON LED bands, Hyeonwoo International Inc.) with different colors on their wrists. General purified water (hardness: 30) as drinking water was supplied to the control group and mineral water (Aribio Co., Ltd.) having a hardness of 700 was supplied to the experimental group. Here, 30 runners in the experimental group supplemented mineral water of hardness 700 in each water bottle or water bag at every water supply support section.

The present study was designed with a randomized block design. As for blocking variables, the systolic blood pressure was set within an error range, leading to no difference between groups. The systolic and diastolic blood pressures were measured using a mercury blood pressure meter (Spirit, Chinkou Medical Instrument, Taiwan) prior to departure and at 50 km and 100 km (total three times). The water supply support points were a total of six sites including the starting point, and mineral water with 700 hardness and general water (hardness 30) were supplied to all of the subjects. For verification of the difference in blood pressure change by section (0 km, 50 km, 100 km) between the experimental group and the control group, the descriptive statistics of the collected data were calculated using SPSS 16.0 for Windows (Chicago, IL, USA) and then, the interaction effect was verified through the two-way mixed model multivariate validation [2 (groups; experimental group, control group) x 3 (repetition; 0 km, 50 km, 100 km) two mixed design ANOVA]. Comparison verification was conducted to verify the difference in change between time periods. The significance level was set to $\alpha$ = 0.05.

## Results

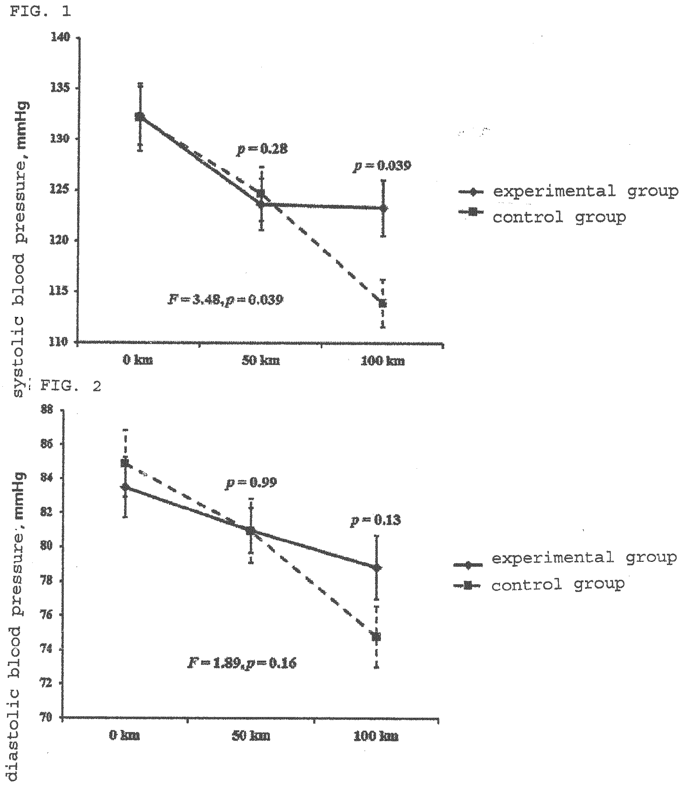

As shown in Figure 1, there was a significant interaction effect according to the exercise distance (0 km, 50 km, 100 km) in the systolic blood pressure between the experimental group and the control group (F = 3.48, $p$ = 0.039). At the final 100 km, the experimental group consuming high-hardness mineral water showed a normal blood pressure range, but the control group showed a sharp decrease ($p$ = 0.039). As shown in FIG. 2, the diastolic blood pressure was maintained at the normal blood pressure level in the experimental group, and the diastolic blood pressure of the control group was lower than that of the experimental group at the final 100 km, and the width of the decrease was also larger ($p$ = 0.13; FIG. 2).

A rapid decrease in blood pressure during exercise, particularly at the time of exercises requiring much physical exertion, such as a marathon and an ultramarathon, is extremely dangerous, and in severe cases, it causes heart failure, leading to death. The above results show that such a rapid decrease in blood pressure can be prevented through the intake of high-hardness of mineral water.

## References (incorporated herein by reference)

1. Chen, Chao-Tin and Bonham (2010). Postexercise hypotension: central mechanisms. Exercise and Sports Science Reviews, 38(3), 122-127.
2. Holtzhausen, Lucy-May and Noakes, T.D. (1995). The prevalence and significance of post-exercise (postural) hypotension in ultramarathon runners. Medicine and Science in Sports and Exercise, 27(12), 1595-1601.
3. MacDonald, J.R. (2002). Potential causes, mechanisms, and implications of post exercise hypotension. Journal of Human Hypertension, 16, 225-236.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

**Claims**

1.  A composition for preventing or alleviating a decrease in blood pressure containing mineral water having a hardness value of 100-2000 prepared from salty underground water or deep sea water as an active ingredient.

2.  The composition of claim 1, wherein the mineral water contains 15-500 mg/ℓ magnesium, 5-170 mg/ℓ calcium and 4.5-150 mg/ℓ potassium.

3.  The composition of claim 1, wherein the mineral water has a hardness value of 100-1200.

4.  The composition of claim 3, wherein the mineral water contains 15-300 mg/ℓ magnesium, 5-102 mg/ℓ calcium and 4.5-90 mg/ℓ potassium.

5.  The composition of claim 1, wherein the decrease in blood pressure is a decrease in blood pressure during or after exercise.

6.  The composition of claim 1, wherein the composition is drinking water, tea, a sports drink, an isotonic drink or an energy drink.

7.  The composition of claim 1, wherein the mineral water is prepared to have a hardness of 100-2000 by mixing desalted water, which is obtained by performing a desalting treatment on salty underground water or deep sea water, with: (i) a mineral concentrate obtained by separating calcium salt crystals and salt from concentrated water obtained by performing a desalting treatment on salty underground water or deep sea water; and (ii) calcium salts obtained by removing, from the calcium salt crystals separated from the concentrated water, salt and calcium carbonate attached to the calcium salt crystals, and wherein the mineral concentrate is obtained by filtering through a filter having a physical adsorbent, re-filtering the filtered mineral concentrate through a filter having a physical adsorbent, and then filtering the re-filtered mineral concentrate through a hollow fiber membrane filter having a plurality of pores.

8.  A composition containing the composition of any one of claims 1 to 7 as an active ingredient for preventing or alleviating blood pressure decrease-associated symptoms caused by a decrease in blood pressure, the symptoms being selected from the group consisting of dizziness, headache, fainting, wobbling, nausea, numbness, systemic weakness, temporary visual and hearing impairment, nervous breakdown, constipation, insomnia, slow pulse, pale skin, shock, and arrhythmia.

9.  A method for preventing or alleviating a decrease in blood pressure comprising a step of administering a composition to a subject in need thereof, the composition containing mineral water having a hardness value of 100-2000 prepared from salty underground water or deep sea water as an active ingredient.

10. A method for preventing or alleviating blood pressure decrease-associated symptoms caused by a decrease in blood pressure comprising a step of administering a composition to a subject in need thereof, the composition containing mineral water having a hardness value of 100-2000 prepared from salty underground water or deep sea water as an active ingredient, the symptoms being selected from the group consisting of dizziness, headache, fainting, wobbling, nausea, numbness, systemic weakness, temporary visual and hearing impairment, nervous breakdown, constipation, insomnia, slow pulse, pale skin, shock and arrhythmia.

FIG. 1

FIG. 2

## INTERNATIONAL SEARCH REPORT

International application No.

**PCT/KR2015/011492**

| A. CLASSIFICATION OF SUBJECT MATTER |
|---|
| *A61K 33/06(2006.01)i, A61K 33/14(2006.01)i, A61P 9/12(2006.01)i* |
| According to International Patent Classification (IPC) or to both national classification and IPC |

| B. FIELDS SEARCHED |
|---|
| Minimum documentation searched (classification system followed by classification symbols) |
| A61K 33/06; A23L 1/304; C02F 1/28; A23L 1/30; C02F 1/68; A61K 35/02; A61K 33/14; A61P 9/12 |
| Documentation searched other than minimum documentation to the extent that such documents are included in the fields searched<br>Korean Utility models and applications for Utility models: IPC as above<br>Japanese Utility models and applications for Utility models: IPC as above |
| Electronic data base consulted during the international search (name of data base and, where practicable, search terms used)<br>eKOMPASS (KIPO internal) & Keywords: saline ground water, deep sea water, mineral water, blood pressure reduction and prevention, low blood pressure |

| C. | DOCUMENTS CONSIDERED TO BE RELEVANT | |
|---|---|---|
| Category* | Citation of document, with indication, where appropriate, of the relevant passages | Relevant to claim No. |
| A | KR 10-2014-0052389 A (ARIBIO INC.) 07 May 2014<br>See abstract; and claims 1-9. | 1-8 |
| A | JP 2007-165990 A (OHASHI, Hiromasa) 05 July 2007<br>See abstract; and claims 1-2. | 1-8 |
| A | RYLANDER, R. et al., "Mineral Water Intake Reduces Blood Pressure Among Subjects with Low Urinary Magnesium and Calcium Levels", BMC Public Health, 2004, vol. 4, paper no. 56, inner pages 1-5<br>See abstract. | 1-8 |
| A | KR 10-2014-0010654 A (ARIBIO INC.) 27 January 2014<br>See abstract; and claims 1-9. | 1-8 |
| A | JP 2003-063969 A (GOSHU YAKUHIN KK.) 05 March 2003<br>See abstract; and claims 1-4. | 1-8 |

☐ Further documents are listed in the continuation of Box C. ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 04 MARCH 2016 (04.03.2016) | **04 MARCH 2016 (04.03.2016)** |

| Name and mailing address of the ISA/KR | Authorized officer |
|---|---|
| Korean Intellectual Property Office<br>Government Complex-Daejeon, 189 Seonsa-ro, Daejeon 302-701,<br>Republic of Korea | |
| Facsimile No. 82-42-472-7140 | Telephone No. |

Form PCT/ISA/210 (second sheet) (January 2015)

## INTERNATIONAL SEARCH REPORT

| International application No. |
| --- |
| **PCT/KR2015/011492** |

**Box No. II     Observations where certain claims were found unsearchable (Continuation of item 2 of first sheet)**

This international search report has not been established in respect of certain claims under Article 17(2)(a) for the following reasons:

1. ☒ Claims Nos.: **9-10**
   because they relate to subject matter not required to be searched by this Authority, namely:

   Claims 9 and 10 pertain to a method for treatment of the human body by therapy, and thus pertain to subject matter on which the International Searching Authority is not required to carry out an international search under the provisions of PCT Article 17(2)(a)(i) and PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III     Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**   ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**
Information on patent family members

International application No.

**PCT/KR2015/011492**

| Patent document cited in search report | Publication date | Patent family member | Publication date |
|---|---|---|---|
| KR 10-2014-0052389 A | 07/05/2014 | EP 2912956 A1<br>US 2015-0290241 A1<br>WO 2014-065550 A1 | 02/09/2015<br>15/10/2015<br>01/05/2014 |
| JP 2007-166990 A | 05/07/2007 | NONE | |
| KR 10-2014-0010654 A | 27/01/2014 | NONE | |
| JP 2003-063969 A | 05/03/2003 | NONE | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHEN ; CHAO-TIN ; BONHAM.** Postexercise hypotension: central mechanisms. *Exercise and Sports Science Reviews,* 2010, vol. 38 (3), 122-127
- **HOLTZHAUSEN ; LUCY-MAY ; NOAKES, T.D.** The prevalence and significance of post-exercise (postural) hypotension in ultramarathon runners. *Medicine and Science in Sports and Exercise,* 1995, vol. 27 (12), 1595-1601

- **MACDONALD, J.R.** Potential causes, mechanisms, and implications of post exercise hypotension. *Journal of Human Hypertension,* 2002, vol. 16, 225-236